(19) Europäisches Patentamt
European Patent Office
Office européen des brevets

(11) Numéro de publication: **0 259 234 A1**

(12) **DEMANDE DE BREVET EUROPEEN**

(21) Numéro de dépôt: **87420219.5**

(22) Date de dépôt: **12.08.87**

(51) Int. Cl.⁴: **C 07 D 405/06**
C 07 D 249/08
// C07D303/18, C07C43/178, C07D307/20

(30) Priorité: **22.08.86 FR 8612096**

(43) Date de publication de la demande: **09.03.88 Bulletin 88/10**

(84) Etats contractants désignés: **AT BE CH DE ES FR GB GR IT LI LU NL SE**

(71) Demandeur: **RHONE-POULENC AGROCHIMIE**
**14-20, rue Pierre Baizet**
**F-69009 Lyon (FR)**

(72) Inventeur: **Corbet, Jean Pierre**
**7 Chemin de Charrière blanche**
**F-69130 Ecully (FR)**

**Mas, Jean Manuel**
**1 Rue du Tonkin**
**F-69100 Villeurbanne (FR)**

(74) Mandataire: **Ranguis, Patrick Frédéric et al**
**RHONE POULENC AGROCHIMIE Service DPI B.P. 9163**
**F-69263 Lyon Cédex 09 (FR)**

(54) **Procédé de préparation de composés à groupe triazole et tétrahydrofuranne, nouveaux composés utilisables pour la préparation des dits composés.**

(57) L'invention concerne un procédé de préparation des composés 2-(1-triazolyl) méthyl 2-(m-, p-dichlorophényl) 5-$OR_1$ tétrahydrofuranne, $R_1$ étant un radical alkyle inférieur éventuellement substitué, au moyen des étapes suivantes :

a) Isomérisation du composé de formule :
(m-,p-di Cl)Ph - C(OH)($CH_2$Cl)-CH = CH-$CH_2$-$OR_1$

b) Greffage d'un noyau triazole sur le composé obtenu de formule :
(m-, p-di Cl)Ph - C(OH)($CH_2$Cl)-CH = CH-$CH_2$-$OR_1$

c) Cyclisation du composé obtenu au b).

EP 0 259 234 A1

**Description**

Procédé de préparation de composés à groupe triazole et tétrahydrofuranne, nouveaux composés utilisables pour la préparation des dits composés

L'invention concerne un procédé de synthèse de composés de formule I dans laquelle :
$R_1$ représente l'atoe d'hydrogène ou un radical alkyle inférieur, cycloalkyle inférieur, aryle (notamment phényle), aralkyle (notamment benzyle), ces divers radicaux pouvant être éventuellement substitués par un ou plusieurs atomes ou radicaux tels que les atomes d'halogènes, les radicaux alkoxy inférieur, aryloxy, hydroxy,
$R_2$, $R_3$, $R_4$, identiques ou différents, représentent l'atome d'hydrogène, ou un radical alkyle inférieur, cycloalkyle inférieur éventuellement substitué par un ou plusieurs atomes ou radicaux tels que les atomes d'halogènes, les radicaux alkoxy inférieurs, aryloxy,hydroxy.
X est un atome d'halogène, de préférence le fluor, le brome ou le chlore, ou un groupe alkyle ou alkoxy ayant de 1 à 12 atomes de carbone, de préférence de 1 à 4 atomes de carbone, et étant éventuellement mono ou polyhalogéné (groupement $CF_3$ notamment), ou un groupe cyano dans le cas où $R_3$ et/ou $R_4$ correspondent à l'atome d'hydrogène,
n est un nombre entier, positif ou nul, inférieur à 6, de préférence égal à 2, étant entendu que lorsque n est supérieur à 1, les substituants X peuvent être soit identiques, soit différents,
m = 0 ou 1,
W représente un groupe trivalent constitué soit d'un groupe =CH- soit d'un atome d'azote =N-,
Le terme "inférieur" qualifie un radical organique comportant au plus six atomes de carbone. Ce radical peut être linéaire ou ramifié.

L'invention concerne également les composés nouveaux obtenus lors des différentes étapes du procédé et leur utilisation pour la synthèse des composés de formule I.

Les composés de formule I sont des produits fongicides bien connus. Ils sont notamment décrits dans les demandes de brevet européen nº 0 121 979 et/ou 0 151 084.

Un but de la présente invention est de fournir un procédé de préparation des tétrahydrofurannes à groupes triazole avec un rendement et des conditions réactionnelles améliorés.

Le procédé selon l'invention est caractérisé en ce qu'il consiste dans les étapes suivantes :
isomérisation des composés de formule IV ou IVa dans laquelle Hal correspond à un atome d'halogène et X, n, m, $R_1$ à $R_4$ ont la même signification que pour la formule I, en présence d'un catalyseur, en phase homogène ou hétérogène, de manière à conduire aux composés de formule III ou IIIa

Cette étape étant suivie :
soit du greffage d'un noyau imidazole ou triazole de manière à obtenir un composé de formule II dans laquelle X, n, m, $R_1$ à $R_4$, W ont la même signification que dans la formule I puis de la cyclisation en milieu acide de manière à obtenir un composé de formule I,
soit, dans le cas du composé de formule III, de la cyclisation en milieu acide de manière à obtenir un composé de formule V dans laquelle X, m, n, $R_1$ à $R_4$, ont la même signification que dans la formule I puis du greffage d'un noyau imidazole ou triazole en présence d'un accepteur d'acide de manière à obtenir le composé de formule I.

Bien que les étapes de greffage et de cyclisation puissent être interverties dans le cas du composé de formule III, il est toutefois préférable de procéder dans l'ordre suivant :
a) isomérisation, b) greffage, c) cyclisation.

Aussi dans la description qui va suivre, sauf si c'est précisé, décrira-t-on les différentes étapes dans cet ordre. Mais il est évident que les conditions opératoires sont identiques lorsque l'ordre des étapes b) et c) est inversé bien que cette voie soit moins avantageuse.

Parmi les catalyseurs utilisables pour assurer l'isomérisation, on citera avantageusement les métaux de transition suivant : ruthénium, cobalt, palladium, nickel, rhodium, iridium, platine.

Ces catalyseurs peuvent fonctionner sous forme hétérogène à l'état métallique, et dans ce cas ils sont déposés sur un support inerte approprié tel que le noir de charbon.De préférence ils peuvent également fonctionner en catalyse homogène sous la forme de complexes comportant outre le métal de transition, un ou plusieurs ligands appropriés (phosphine, carbonyle) et une ou plusieurs molécules d'hydrures.

De façon inattendue cette réaction s'effectue avec d'excellents rendements, notamment, dans le cas de catalyseurs à base de rhuthénium alors que l'homme de métier aurait pu s'attendre normalement à une réaction parasite résultant du déplacement de la double liaison vers le carbone portant le groupement hydroxy (isomérisation de l'alcool allyllique) ou époxy.

La réaction peut être effectuée en masse ou en présence d'un solvant protique ou aprotique.

Parmi les solvants aprotiques on peut citer les hydrocarbures aliphatiques saturés tels que le n-pentane, l'isopentane, le 2-méthylhexane, le 2,2,5-triméthylhexane, les hydrocarbures aromatiques tels que le benzène, le toluène, l'éthylbenzène, les éthers aliphatiques saturés tels que le tétrahydrofuranne, l'isopentyléther, les éthers aromatiques tels que le benzyléthyléther, les cétones aliphatiques saturées ou aromatiques comme la méthyl-éthylcétone, la méthyl-isobutylcétone, l'acétophénone, les hydrocarbures halogénés saturés aliphatiques ou aromatiques comme le fluorobenzène, le 1-chloro-2-méthylpropane, le chlorure d'isobutyle, les esters aliphatiques saturés ou aromatiques comme l'isobutyrate d'isobutyle, l'acétate d'éthyle, le benzoate de méthyle. Tous ces solvants peuvent être présents seuls ou en mélange.

Lorsque l'on utilise un solvant, on préfèrera travailler dans un milieu dilué comportant 1 % à 70 % en poids de composé de formule III ou IIIa ou V par rapport à la solution totale.

L'accepteur d'acide est au moins présent en quantité stoechiométrique en équivalent d'atome d'hydrogène labile du triazole ou imidazole. En général un rapport en équivalent molaire compris entre 1 et 2,5 sera satisfaisant.

Il est évident que le dérivé salifié du triazole ou imidazole peut être préparé à part et dans ce cas la présence d'un accepteur d'acide n'est pas requise pour la réaction de greffage. Cette préparation s'éffectue en milieu anhydre ou non anhydre dans un solvant dans les mêmes conditions opératoires que celles décrites dans le cas de la formation in situ du dérivé triazole ou imidazole salifié.

En ce qui concerne l'étape de greffage il est avantageux, et ceci constitue une des caractéristiques de l'invention de passer par un intermédiaire époxyde de formule IIIa, dans laquelle X, n, m, $R_1$ à $R_4$, ont la même signification que dans la formule 1, soit par action sur le composé de formule III d'une base alcoolique comme la potasse ou la soude méthanolique (généralement la réaction s'éffectue à température comprise entre -10° C et 50° C en présence de 1 à 2 équivalent molaire d'alcoolate par mole de composé de formule III), soit par isomérisation dans les conditions opératoires identiques à celles de la chlorhydrine de formule IV de l'époxyde de formule IVa obtenu par traitement basique du composé de formule IV dans des conditions identiques à celles indiquées précédemment.

Le produit intermédiaire de formule IIIa peut être isolé par distillation ou au contraire être soumis directement à l'étape proprement dite de greffage du noyau imidazole ou triazole. Dans ce cas la réaction peut être effectuée à des températures inférieures à 130° C et supérieures à 50° C, éventuellement dans un solvant approprié de préférence polaire comme le diméthylformamide ou le diméthylsulfoxyde ou les alcools de $C_1$ à $C_4$.

Le rapport molaire dérivé salifié du triazole ou imidazole par rapport au composé de formule (IIIa) est compris comme précédemment entre 1,05 et 1,5. Comme précédemment le dérivé salifié de l'imidazole ou triazole peut être préparé in situ par action de la base correspondante.

En ce qui concerne l'etape de cyclisation, la réaction s'effectue en milieu acide.

L'acide catalyseur mis en oeuvre dans cette réaction peut être un acide protique ou aprotique, soluble ou insoluble. Comme acides protiques on peut citer les acides chlorhydrique, sulfurique, trifluoroacétique, perchlorique, benzène-sulfonique, toluène-sulfonique, méthane-sulfonique. Comme acides aprotiques on peut citer les acides de Lewis tels que $BF_3$, $AlCl_3$ et $SnCl_4$. On peut également utiliser les résines solides comme les résines sulfoniques.

On utilisera de préférence de 0,1 à 2 équivalents molaire d'acides par mole de composé de formule II ou III.

La réaction s'effectue ordinairement par simple chauffage des réactifs indiqués. La température est généralement comprise entre 10° C et 100° C ou si il y a un solvant entre 10° C et la température d'ébulition du solvant considéré. Comme solvant on peut notamment utiliser un hydrocarbure aliphatique, alicyclique ou aromatique, halogéné ou non, un éther, ou un alcool tel que celui de formule $R_1OH$.

Une variante particulièrement préférée du procédé selon l'invention consiste afin d'atteindre les composés de formule I dans laquelle $R_1$ représente un radical alkyle inférieur, cycloalkyle inférieur, aryle, aralkyle, lesdits radicaux étant substitués comme c'est indiqué lors de la définition générale du radical $R_1$ au début de la description, consiste à faire réagir un composé de formule II ou III dans laquelle $R_1$ correspond à l'atome d'hydrogène ou un radical alkyle inférieur, cycloalkyle inférieur, aryle, aralkyle avec un composé de formule R OH dans laquelle R correspond à un radical alkyle inférieur, cycloalkyl inférieur, aryle, aralkyle, lesdits radicaux étant substitués comme c'est indiqué lors de la définition générale du radical $R_1$ au début de la description, en présence d'un catalyseur acide précité et dans les conditions opératoires énumérées déjà lors de la description de l'étape de cyclisation.

Cette réaction conduit avec un excellent rendement au produit souhaité.

En général la réaction s'effectue en excès molaire de composé de formule $R_5OH$ de préférence de 1,05 à 10 et avantageusement de 2 à 6.

Dans le cas où $R_1$ est l'atome d'hydrogène l'étape d'isomérisation conduit si l'on part du composé de formule IV ou IVa à la cyclisation, les étapes d'isomérisation et de cyclisation sont alors enchainées. L'étape de greffage est effectuée ensuite.

Quelque soit le procédé utilisé, le composé de formule I formé en fin de réaction est isolé du milieu réactionnel par tout moyen en soi connu tel que, par exemple par distillation du solvant, ou par cristallisation du produit dans le milieu réactionnel, ou par filtration, puis si nécessaire, ce composé est purifié par des méthodes usuelles telles que recristallisation dans un solvant approprié.

L'invention a également pour objet les composés de formule II, III, IIIa, IV, IVa, VI, VII, VIII dans lesquelles X, $R_1$ à $R_4$, m, n, Hal, W ont la même signification que dans le composé de formule I. Ces composés sont utilisés pour la préparation du composé de formule I de même que le composé de formule V.

Les exemples suivants illustrent l'invention :

Exemple 1 : Préparation du 2-(2,4-dichloro 1-phényl) 2-(1-triazolylméthyl) 5-trifluoroéthoxy tétrahydrofuranne

Dans un ballon de 10 ml sous atmosphère inerte on introduit 0,984 g (3mmoles) de 2-(2,4-dichloro 1-phényl) 5-méthoxy 1-triazolyl 4-pentène 2-ol et 3 ml de trifluoroéthanol. On introduit à 75° C environ de l'HCl gazeux. Le méthanol formé est éliminé par distillation. Après 10h, on refroidit, neutralise, extrait, filtre. Le résidu (0,68 g ; 1,71 mmole) fond à 162° C.

Soulignons que la nomenclature employée est française mis à part le fait que la place des substituants est indiquée en amont desdits substituants.

Parmi les solvants protiques on peut citer les alcools aliphatiques saturés ou aromatiques tels que le méthanol, l'isopropanol, le phénol, les acides aliphatiques saturés ou aromatiques comme l'acide acétique, l'acide benzoïque. On peut également utiliser un acide minéral en mélange avec un solvant organique précité dans lequel il est miscible par exemple l'acide chlorhydrique dans le méthanol.Dans ce cas on obtient directement les produits de formule V. Tous ces solvants peuvent être présents en mélange ou seuls.

Parmi ces solvants on préfère les hydrocarbures aromatiques ou les alcools inférieurs aliphatiques saturés. Si la réaction s'effectue une présence d'un solvant, la dilution du produit réactionnel sera notamment comprise entre 0,5 % et 99 % en poids par rapport au poids total de la solution et de préférence entre 5 et 50 %.

La quantité de catalyseur mise en jeu sera de préférence comprise entre 0,0005 et 0,1 mole par mole de produit de formule IV ou IVa.

La température de la réaction peut varier dans une large gamme par exemple entre -20° C et la température de décomposition du catalyseur. Néanmoins un bon compromis entre la durée de la réaction et des conditions opératoires industrielles conduisent à préférer une gamme de température allant de 0°C à 100°C avantageusement entre 10°C et 80°C.

La pression est généralement comprise entre 1 et 10 atmosphères (0,1 à 1 MPa).

Dans le cas où les groupements $R_3$ et $R_4$ correspondent tous les deux à l'atome d'hydrogène une voie de synthèse préférée du composé de formule IV ou IVa consiste à hydrogèner le composé de formule VI, dans laquelle X,n, m, $R_1$, $R_2$, Hal ont la même signification que dans la formule I ou IV, par une quantité équimolaire d'hydrogène au moyen d'un catalyseur approprié éventuellement empoisonné choisi parmi les catalyseurs suivants : palladium, ruthénium, nickel de Raney, platine rhodium et de préférence le palladium éventuellement empoisonné (pyridine, quinoléine) qui donne spécifiquement l'oléfine cis.

Comme précédemment cette réaction peut être effectuée en phase homogène ou hétérogène.

De préférence on choisira le palladium à l'état métallique déposé sur un support inerte tel que le noir de carbone, le carbonate de calcium ou le sulfate de baryum.

La réaction d'hydrogénation bien que cela ne soit pas indispensable peut avantageusement être effectuée en solvant polaire protique ou aprotique identique à ceux utilisés pour l'étape d'isomérisation mis à part que pour le nickel de Raney les hydrocarbures halogénés ne peuvent être utilisés.

La dilution du composé de formule VI est de préférence comprise entre 1 et 80 % en poids ou mieux 5 % et 40 % par rapport au poids total de solution.

De même si la proportion molaire de catalyseur par rapport au composé de formule VI peut varier de manière considérable il sera préférable pour des raisons industrielles évidentes d'utiliser le catalyseur dans une proportion molaire comprise entre 0,01 et 0,5 % par rapport au composé de formule VI.

La réaction s'effectue à des températures comprises entre - 20° C et 150° C, de préférence entre 10 et 80° C et à une pression généralement comprise entre 1 et 10 atmosphère (0,1 MPa et 1 MPa).

Le composé de formule VI peut être obtenu par addition de l'organomagnésien de formule

$R_2$-($OR_1$)CH-C=C-Mg-Br VII

sur une halogénoacétophénone de formule

(X)n-Ph($CH_2$)m-CO$CH_2$Hal VIII

La réaction peut s'éffectuer par exemple dans le tétrahydrofuranne de manière connue ou dans un mélange d'hydrocarbure tel que le toluène et d'éther tel que le tétrahydrofuranne.

Dans le cas où au moins un des groupes $R_3$ ou $R_4$ ne correspondent pas à l'atome d'hydrogène, il est possible d'utiliser le voie de synthèse suivante ; addition d'un organomagnésien de formule $R_4$MgX sur les composés de formule générale VI, en présence ou non d'iodure de cuivre suivie éventuellement de l'addition d'un halogénure d'alkyle $R_3$X dans un solvant tel que le tétrahydrofuranne. Dans le cas où l'on s'arrête à l'addition de $R_4$MgX il est bien évident que l'on fait suivre cette étape d'addition d'une hydrolyse.

Le composé IVa est obtenu par action d'une base alcoolique (soude,potasse méthanolique) sur le composé IV (généralement la réaction s'éffectue à température comprise entre -10°C et 50°C en présence de 1 à 2 équivalent molaire d'alcoolate par mole de composé de formule IV).

L'étape de greffage est avantageusement effectuée en présence d'un accepteur d'acide, en milieu anhydre ou non anhydre, dans un solvant, inerte dans les conditions de la réaction, généralement entre 50 et 180°C et de préférence à une température voisine de la température d'ébullition du solvant. Comme accepteurs d'acide, on peut mentionner des bases minérales comme par exemple la soude ou la potasse, les carbonates de métaux alcalins ou de métaux alcalinoterreux, des bases azotées comme la triéthylamine, des amines quaternaires comme le tétrabutylammonium hydroxyde, ou des hydroxydes de phosphonium. Comme solvants on utilise avantageusement des solvants aprotiques polaires, tels que par exemple, le diméthylformamide, le diméthylacétamide, le diméthylsulfoxyde, l'acétone, la méthyl-éthylcétone, la méthyl-isobutylcétone, l'acétonitrile, la N-méthylpyrrolidone ou des solvants protiques polaires tels que le méthanol ou le propanol. Si désiré, cette réaction peut être effectuée en présence d'un catalyseur approprié. Comme catalyseur utilisable, on peut mentionner des catalyseurs de transfert de phases, tels que par exemple, des dérivés d'ammonium quaternaires comme le chlorure de tétrabutylammonium.

La réaction de greffage est de préférence effectuée en excès molaire de dérivé de triazole ou imidazole de préférence compris entre 1,05 et 1,5.

Rendement / au produit de départ : 57,2 %.

Exemple 2 : Préparation du 2-(2,4-dichloro 1-phényl) 5-méthoxy 1-triazolyl 4-pentène 2-ol

Dans un ballon de 10 ml sous atmosphère inerte on introduit 0,828 g (11,9 mmoles) de 1,2,4-triazole, 0,54 g de méthylate de sodium (0,54 g ; 10,0 mmoles) et 2,5 ml de N,N-diméthylformamide sec. Après 15 minutes on ajoute 2,59 g (10,0 mmoles) de 1-(2,4-dichloro 1-phényl) 1-(3-méthoxy 2-propène 1-yl) oxiranne (isomère cis et trans) dans 2,5 ml de DMF anhydre. Après 5 h à 118°C, on refroidit, ajoute 25 ml de $CH_2Cl_2$, lave à l'eau, extrait, sèche. L'extrait sec est un résidu huileux : 3,37 g. Après purification on obtient 2,14 g (6,52 mmoles) d'un produit pur huileux.
Rendement : 65 % / produit de départ.

Exemple 3 : Préparation du 1-(2,4-dichloro 1-phényl) 1-(3-méthoxy 2-propène 1-yl) oxiranne (isomère cis et trans)

Une solution de 1,19 g (4,03 mmoles) de 1-chloro2-(2,4-dichloro 1-phényl) 5-méthoxy 4-pentène 2-ol est introduite dans un ballon de 25 ml sous atmosphère inerte avec 2ml de méthanol et 46,27 mg (0,04 mmoles) de $RuH_2$ $(PPH_3)_4$ pendant 1 h à 50°C. On refroidit, concentre, filtre le catalyseur. On obtient une huile jaune (1,19 g ; 4,03 mmoles).
Rendement : 100 % / au produit de départ.

Example 4 : Préparation du 1-chloro 2-(2,4-dichloro 1-phényl) 5-méthoxy 4-pentène 2-ol

Dans un ballon de 11 sous atmosphère inerte, on introduit 40,2 g (136,93 mmoles) de 1-chloro 2-(2,4-dichloro 1-phényl) 5-méthoxy 3-pentyne 2-ol, 300 ml de méthanol et 218,3 mg (0,102 miliatome gramme) de palladium à 5 % sur charbon. On introduit l'hydrogène . Après 2 h 30 à 22°C on chasse l'hydrogène, filtre, concentre.
Rendement : 98 % de résidu huileux.

Exemple 4 : Préparation du 1-chloro 2-(2,4-dichloro 1-phényl) 5-méthoxy 3-pentyne 2-ol

Dans un ballon de 100 ml sous atmosphère inerte on introduit 4 g (0,164 mole) de magnésium et 8 ml de THF. On ajoute 16,34 g (0,149 mole) de bromoéthane dans 60 ml de THF en maintenant la température à 25°C pendant 1 h. A la fin de la coulée on ajoute 40 ml de THF. Le magnésium ainsi obtenu est filtré sous atmosphère inerte puis coulé sur une solution de 10,57 g (0,15 mole) de 3-méthoxy 1-propyne dans 50 ml de THF placé dans un ballon de 250 ml sous atmosphère inerte. La température est maintenue à 20-25°C. A la fin de la coulée on ajoute 22,35 g (0,1 mole) de 2,4,2'- trichloroacétophénone dans 25 ml de THF. La température est maintenue à 22-24°C. Ein fin d'addition on ajoute 9 g (0,15 mole) d'acide acétique puis concentre jusqu'à un volume de 100 ml. On ajoute alors 75 ml de méthylcyclohexane, lave, concentre, recristallise dans le cyclohexane. On obtient 21,45 g (73,16 mmoles) de produit, fondant à 98°C, sous la forme de cristaux blancs.
Rendement : 73 %.
Le 3-méthoxy 1-propyne est préparé selon W. REPPE, Annalen, 596, 74, 1955.

Exemple 6 : Préparation du 2-(2,4-dichloro 1-phényl) 2-(1-chlorométhyl) 5-méthoxy tétrahydrofuranne

Dans un ballon de 25 ml sous atmosphère inerte, on introduit 0,860 g (2,9 mmole) de 1-chloro 2-(2,4-dichloro 1-phényl) 5-méthoxy 4-pentène 2-ol, 5 ml de toluène sec et 0,05 g (0,29 mmole) d'acide p-toluène sulfonique. Après 30 minutes le milieu est refroidi, lavé à l'eau, séché, concentré. On obtient 0,6 g d'un résidu huileux pur à 95 %.

Exemple 7 : Préparation du 2-(2,4-dichloro 1-phényl) 2-(1-triazolyl méthyl) 5-méthoxy tétrahydrofuranne

Dans un ballon de 10 ml sous atmosphère inerte, on introduit 0,3 g (1 mmole) de 2-(2,4-dichloro 1-phényl) 2-(1-chlorométhyl) 5-métoxy tétrahydrofuranne en solution dans 3 ml de N-méthylpyrrolidone et 0,092 g (1 mmole) de triazolate de sodium. Après 6 h à 160°C on refroidit, on obtient 0,414 g d'un brut dans lequel on dose 74,21 mg de composé.
Rendement : 22, 6%.

Exemple 8 : Préparation du 2-(2,4-dichloro 1-phényl) 2-(1-triazolyl méthyl) 5-hydroxy tétrahydrofuranne

Dans un ballon de 10 ml on introduit 0,131 g de 2-(2,4 -dichloro 1-phényl) 5-méthoxy 1-triazolyl 4-pentène 2-ol (0,4 mole) dans 0,5 ml de N,N-diméthyl formamide et 2 ml d'une solution HCl 2N. Après 2 h 30 à 60°C, le milieu est neutralisé après élimination du surnageant, lavage à l'eau, séchage, on obtient 0,124 g d'un brut réactionnel qui repris à l'éther de pétrole cristallise pour donner après filtration et séchage 0,107 g (0,34 mmole) du dérivé hydroxylé sous forme d'un mélange 80/20 des deux diastéréosimères.

## FORMULES DES COMPOSES

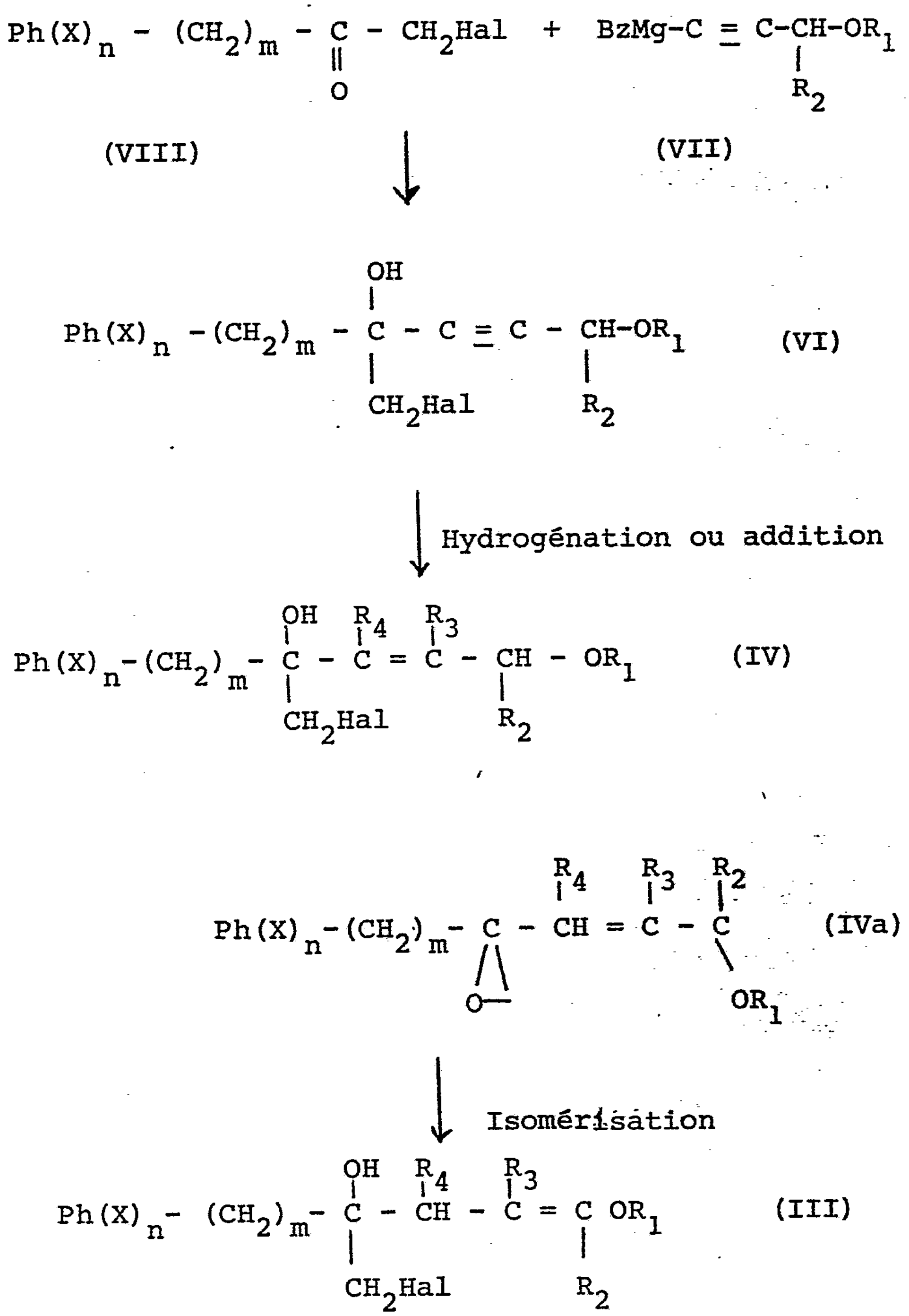

FORMULES DES COMPOSES (suite)

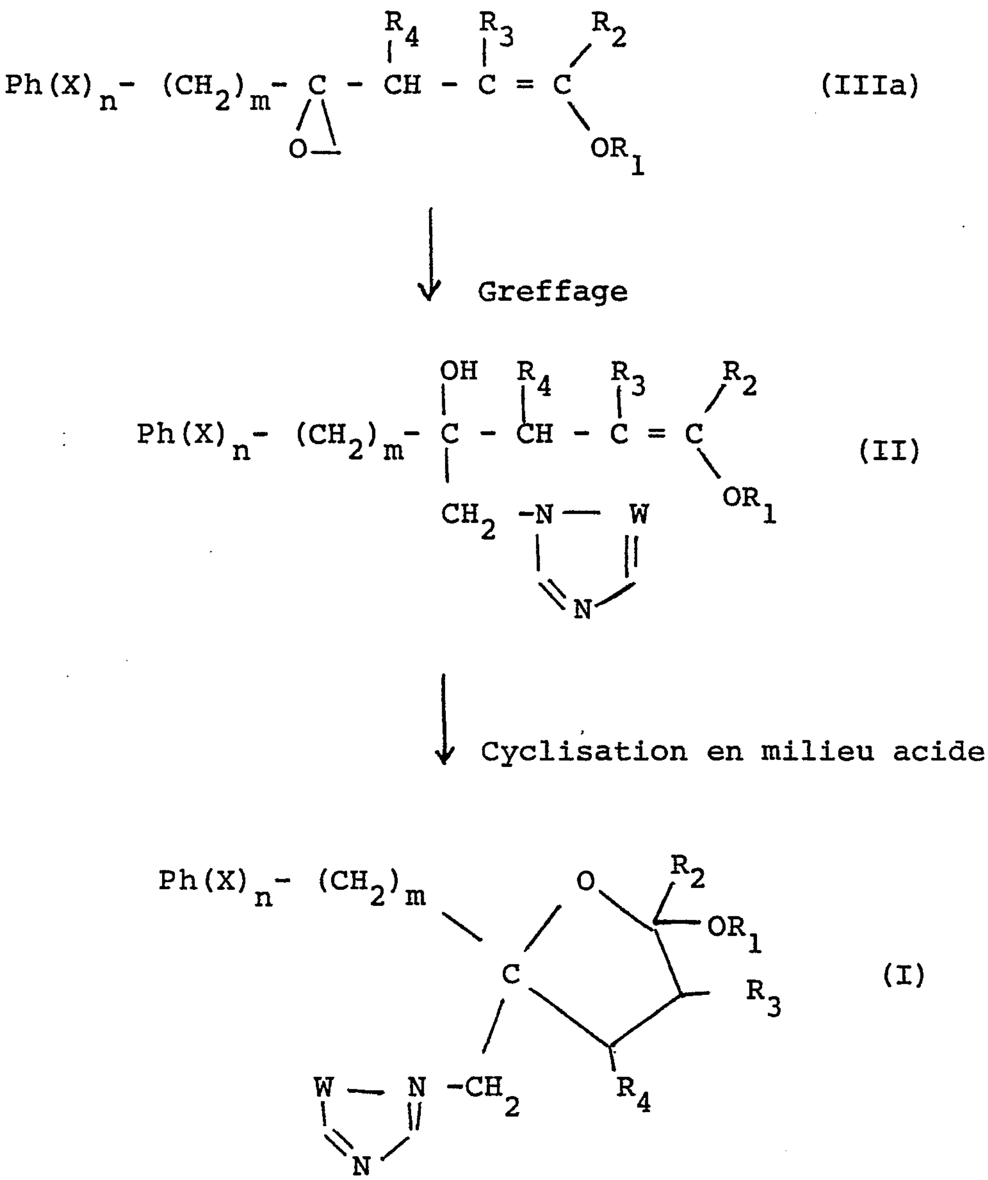


(IIIa)

(II)

(I)

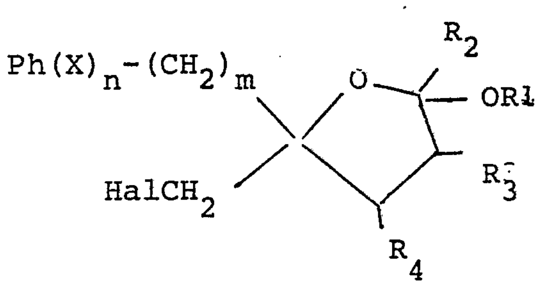

(V)

**Revendications**

1) Procédé de synthèse de composés de formule I dans laquelle :
$R_1$ représente l'atome d'hydrogène ou un radical alkyle inférieur, cycloalkyle inférieur, aryle (notamment phényle), aralkyle (notamment benzyle), ces divers radicaux pouvant être éventuellement substitués par un ou plusieurs atomes ou radicaux tels que les atomes d'halogènes, les radicaux alkoxy inférieur, aryloxy, hydroxy,
$R_2$, $R_3$, $R_4$, identiques ou différents, représentent l'atome d'hydrogène, ou un radical alkyle inférieur, cycloalkyle inférieur éventuellement substitué par un ou plusieurs atomes ou radicaux tels que les atomes d'halogènes, les radicaux alkoxy inférieurs, aryloxy, hydroxy,
X est un atome d'halogène, de préférence le fluor, le brome ou le chlore, ou un groupe alkyle ou alkoxy ayant de 1 à 12 atomes de carbone, de préférence de 1 à 4 atomes de carbone, et étant éventuellement mono ou polyhalogéné (groupement $CF_3$ notamment), ou un groupe cyano dans le cas où $R_3$ et/ou $R_4$ correspondent à l'atome d'hydrogène,
n est un nombre entier, positif ou nul, inférieur à 6, de préférence égal à 2, étant entendu que lorsque n est supérieur à 1, les substituents X peuvent être soit identiques, soit différents,
m = 0 ou 1,
W représente un groupe trivalent constitué soit d'un groupe =CH- soit d'un atome d'azote =N-,
le procédé étant caractérisé en ce qu'il consiste dans les étapes suivantes :
isomérisation des composés de formule IV ou IVa dans laquelle Hal correspond à un atome d'halogène et X, n, m, $R_1$ à $R_4$ ont la même signification que pour la formule I, en présence d'un catalyseur, en phase homogène ou hétérogène, de manière à conduire aux composés de formule III ou IIIa.
Cette étape étant suivie :
soit du greffage d'un noyau imidazole ou triazole de manière à obtenir un composé de formule II dans laquelle X, n, m, $R_1$ à $R_4$, W ont la même signification que dans la formule I puis de la cyclisation en milieu acide de manière à obtenir un composé de formule I, soit, dans le cas du composé de formule III, de la cyclisation en milieu acide de manière à obtenir un composé de formule V dans laquelle X, m, n, $R_1$ à $R_4$, ont la même signification que dans la formule I puis du greffage d'un noyau imidazole ou triazole en présence d'un accepteur d'acide de manière à obtenir le composé de formule I.

2) Procédé selon la revendication 1, caractérisé en ce que le catalyseur d'isomérisation est choisi dans le groupe formé par : le ruthénium, le cobalt, le palladium, le nickel, le rhodium, l'iridium, le platine.

3) Procédé selon la revendication 2, caractérisé en ce que la proportion de catalyseur en mole par rapport au composés de formule IV ou IVa est comprise entre 0,0005 et 0,1.

4) Procédé selon la revendication 1, caractérisé en ce que la réaction d'isomérisation s'effectue en présence d'un solvant et en ce que, de préférence, la quantité de composés de formule IV ou IVa par rapport au solvant est comprise entre 5 et 50 % en poids.

5) Procédé selon la revendication 1, caractérisé en ce que la température de la réaction d'isomérisation est comprise entre 10° C et 80° C.

6) Procédé selon la revendication 1, caractérisé en ce que l'étape de greffage est effectuée au moyen d'un dérivé salifié d'un imidazole ou triazole formé ou non in situ, et en ce que le rapport molaire dudit dérivé par rapport au composé de formule III ou IIIa ou V est compris entre 1,05 et 1,5.

7) Procédé selon l'une des revendications 1 ou 6, caractérisé en ce que la réaction de l'étape de greffage est effectuée en présence d'un solvant et en que la quantité de composés de formule III ou IIIa ou V par rapport au solvant est comprise entre 1 et 70 % en poids.

8) Procédé selon l'une des revendications 1, 6 ou 7, caractérisé en ce que la témpérature de la réaction de l'étape de greffage est voisine de la température d'ébulition du solvant.

9) Procédé selon la revendication 1, caractérisé en ce que la réaction de l'étape de greffage est effectuée en deux demie étapes 1) et 2) :
1) formation d'un composé formule IIIa dans laquelle X, $R_1$ à $R_4$, m, n ont la même signification que pour le composé de formule I par action d'une base alcoolique sur le composé de formule III ou par isomérisation du composé de formule IVa,
2) greffage d'un noyau imidazole ou triazole dans les mêmes conditions opératoires que celles définies aux revendications 6, 7 ou 8.

10)Procédé selon la revendication 9, caractérisé en ce que la réaction avec la base alcoolique est effectuée en présence de 1 à 2 équivalents molaires de base par mole de composé de formule III.

11)Procédé selon la revendication 9 ou 10, caractérisé en ce que la température de la réaction de la demie étape 1) est comprise entre -10° C et 50° C.

12)Procédé selon la revendication 9, caractérisé en ce que la température de la réaction de la demie étape 2) est comprise entre 50° C et 130° C.

13)Procédé selon la revendication 1, caractérisé en ce que la réaction de cyclisation est effectuée en présence de 0,1 à 2 équivalents molaires d'acides par mole composé de formule II.ou III

14)Procédé selon l'une des revendications 1 ou 13, caractérisé en ce que la température de la réaction

est comprise entre 10° C et 100° C ou s'il y a un solvant entre 10° C et la température d'ébulition du solvant considéré.

15)Procédé selon la revendication 1, caractérisé en ce que les composés de formule I dans laquelle $R_1$ représente un radical alkyle inférieur, cycloalkyle inférieur, aryle, aralkyle, substitué comme indiqué à la revendication 1 sont obtenues par réaction d'un composé de formule II ou III dans laquelle $R_1$ correspond à l'atome d'hydrogène ou à un radical alkyle inférieur, cycloalkyle inférieur, aryle, aralkyle avec un composé de formule $R_5OH$ dans laquelle $R_5$ représente un radical alkyle inférieur, cycloalkyle inférieur, aryle, aralkyle substitué comme indiqué à la revendication 1 en présence d'un catalyseur acide et dans les mêmes conditions opératoire que celles indiquées dans les revendications 13 et 14.

16)Procédé selon la revendication 15, caractérisé en ce que le rapport molaire du composé de formule $R_5OH$ par rapport au composé de formule II ou III est compris entre 1,05 et 10.

17)Procédé selon la revendication 1 caractérisé en ce que dans le cas où $R_1$ correspond à l'atome d'hydrogène la cyclisation du composé de formule III ou IIIa est effectuée lors de l'étape d'isomérisation..

18)Utilisation des composés de formules II, III ou IIIa, IV ou IVa, V, VI, VII, VIII, dans lesquelles X, $R_1$ à $R_4$, m, n, Hal,W ont la même signification que dans le composé de formule I comme intermédiaire pour la fabrication des composés de formule I.

19)Composés de formule II, dans laquelle X, $R_1$ à $R_4$, m, n, W ont la même signification que dans le composé de formule I.

Office européen des brevets

# RAPPORT DE RECHERCHE EUROPEENNE

Numero de la demande

EP 87 42 0219

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 151 084 (RHONE-POULENC)<br>* Revendications *<br>--- | 1-19 | C 07 D 405/06<br>C 07 D 249/08 //<br>C 07 D 303/18<br>C 07 C 43/178<br>C 07 D 307/20 |
| D,A | EP-A-0 121 979 (I.C.I.)<br>* Revendications *<br>----- | 1-19 | |
| | | | **DOMAINES TECHNIQUES RECHERCHES (Int. Cl.4)** |
| | | | C 07 D 405/00<br>C 07 D 249/00 |

Le présent rapport a été établi pour toutes les revendications

| Lieu de la recherche | Date d'achèvement de la recherche | Examinateur |
|---|---|---|
| LA HAYE | 26-10-1987 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)